# EUROPEAN PATENT SPECIFICATION

(11) **EP 3 391 853 B1**
(45) Date of publication and mention of the grant of the patent: **20.11.2019**
(21) Application number: 18275052.1
(22) Date of filing: 19.04.2018
(51) Int. Cl.: A61F 2/07

(54) **SUPPORT STRUCTURE FOR SCALLOPED GRAFTS**
STÜTZSTRUKTUR FÜR BOGENFÖRMIGE TRANSPLANTATE
STRUCTURE DE SUPPORT POUR GREFFONS FESTONNÉS

(30) Priority: 19.04.2017 US 201762487108 P
(43) Date of publication of application: 24.10.2018
(73) Proprietor: Cook Medical Technologies LLC, Bloomington, IN 47404 (US)
(72) Inventor: SKENDER, Davorin K., Bloomington, IN 47403 (US); ROEDER, Blayne A., Bloomington, IN 47401 (US)
(74) Representative: Williams Powell

(56) References cited:
- US-A1- 2013 116 775
- US-A1- 2013 289 701
- US-B2- 7 413 573

## Description

### TECHNICAL FIELD

The present disclosure relates to a prosthesis such as a stent graft for deployment in a body vessel, including at arterial branching points.

### BACKGROUND

A stent graft is commonly used to reinforce a weakened area in an artery such as an aneurysm. Blood pressure, among other factors, can cause this weakened area to bulge over time. The bulged area can eventually enlarge and rupture if not treated. The stent graft is designed to seal tightly with the artery above and below the aneurysm to allow the blood to flow through the stent graft without causing the weakened area to bulge. When the aneurysm is located in a region of a body lumen adjacent to a branch vessel, the stent graft may need to accommodate the opening to that vessel so that blood flow is not blocked or obstructed. For example, a stent graft may include fenestrations, branches, bare stents and the like to allow blood to flow into a branch vessel.

US-7,413,573 discloses a stent graft having a scallop with at least three sides and a scallop support structure partially bounded about the perimeter of the scallop and including two longitudinal perimeter support sections and an undulating base.

### SUMMARY

The present invention seeks to provide an improved prosthesis such as a stent graft.

According to an aspect of the present invention, there is provided a stent graft comprising: a substantially tubular graft having a first end and a second end; a scallop cut out of one or both of the first end and the second end, wherein the scallop extends away from an end edge of the substantially tubular graft and has a perimeter defined by graft material, wherein the perimeter has at least three sides including a lateral side having a lateral edge substantially parallel to the end edge and two longitudinal sides extending between the lateral side and the end edge; and a scallop support structure at least partially bounded about the perimeter of the scallop, the scallop support structure comprising at least two substantially longitudinal perimeter support sections, and an undulating lateral base extending between the at least two substantially longitudinal perimeter support sections, wherein the undulating lateral base has at least two undulations; wherein one of the at least two perimeter support sections is attached along and defines one of the two longitudinal sides and another of the at least two perimeter support sections is attached along and defines the other of the longitudinal sides; and wherein the undulating lateral base is disposed along the lateral base of the scallop and , preferably entirely, overlaps the graft material.

There is described a stent-graft having at least one scallop or cut out in an edge of the graft. The scallop has three sides including a lateral side having a lateral edge. A scallop support structure at least partially conforms to the perimeter of the scallop and has an undulating base that extends below the edge of the scallop and has peaks and valleys. The peaks abut the edge of the scallop and the valleys extend below the edge of the scallop. The scallop support structure has at least two substantially longitudinal perimeter support sections, and the undulating lateral base extends between the at least two substantially longitudinal perimeter support sections. The undulating lateral base has at least two undulations. The two perimeter support sections bound the longitudinal sides.

Further aspects of the invention are described here with reference to the drawings.

### BRIEF DESCRIPTION OF THE DRAWINGS

Embodiments of the present invention are described below, by way of example only, with reference to the accompanying drawings, in which:
FIGURE 1 is a perspective view of a stent graft having a scallop-shaped opening at one end;
FIGURE 2 is a front view of the tubular graft material shown in Figure 1;
FIGURE 3 is a support structure for a scallop-shaped opening shown in Figure 1;
FIGURE 4 is a perspective view of a stent graft with another example of a support structure for a scallop-shaped opening in a stent graft;
FIGURE 5 is another example of a support structure for a scallop-shaped opening in a stent graft;
FIGURE 6 is a view of another example of a support structure for a scallop-shaped opening in a stent graft;
FIGURE 7 is a view of another example of a support structure for a scallop-shaped opening in a stent graft;
FIGURE 8 is an example of a setting fixture for manufacturing a support structure;

The drawings described herein are for illustration purposes only and are not intended to limit the scope of the present disclosure in any way.

### DETAILED DESCRIPTION

In the present application, the term "proximal" refers to a direction that is farthest away from the operator when referring to a delivery device, while the term "distal" refers to a direction that is generally closest to the operator using the delivery device. The distal end of the delivery device is that portion of the device that is intended to remain outside of a patient during a procedure. When referring to the prosthesis itself relative to the delivery device, the proximal end of the prosthesis is that part of the prosthesis nearest the proximal end of the delivery device and the distal end of the prosthesis is that end that is closest to the distal end of the delivery device.

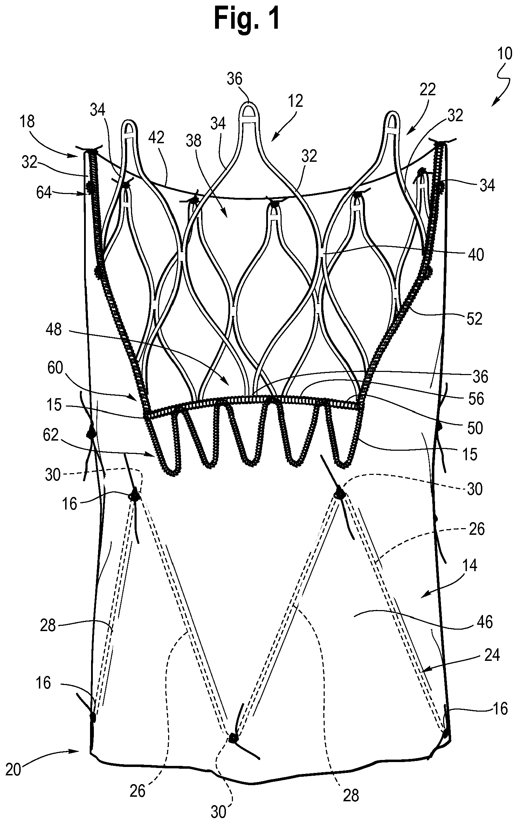

Figure 1 illustrates a portion of a stent graft configured in accordance with a first embodiment of the present disclosure. As shown there, a stent graft 10 includes a stent structure 12, and a tubular graft material 14 attached to the stent structure 12. A suitable stent structure 12 for use in connection with the stent graft 10 described herein may include one or more self-expanding or mechanically-expandable stents or both, and may be deployed according to conventional methodology. A self-expanding stent may be manufactured from stainless steel or a shape-memory alloy, such as nickel titanium alloy (nitinol), cobalt chromium alloy or any other suitable material including cobalt-chromium alloys, amorphous metals, and/or non-metallic materials as would be recognized by one of skill in the art. If the stent comprises a self-expanding material such as nitinol, the stent may be heat-set into the desired expanded state whereby the stent can assume a relaxed radially expanded configuration. Additionally or alternatively, the stent graft 10 may be mechanically expanded, such as through the use of an expandable balloon placed within a lumen of the stent graft 10 and then radially outwardly expanded to thereby expand the stent graft 10.

In one example, the stent structure 12 may include a self-expandable proximal sealing stent 22 to engage a vessel wall when deployed adjacent to a weakened area of the body lumen, or aneurysm. The stent structure 12 may also include one or a series of stents 24 such as z-stents or the like located distal to the proximal sealing stent 22 as shown in Figure 1. The stent structure 12 may be attached to the interior surface of the graft material 14 or it may be exposed on the exterior of the graft material, or both. The stents may all be the same or they may be different. The stent graft 10 may have any desirable design or configuration depending on the particular use, position of placement in a vessel and/or the procedure being performed. In one example, the stent graft 10 resembles the stent grafts disclosed in U.S. Patent Nos. 7,413,573, 8,672,993, and 8,702,780.

As will be apparent from Figure 1, the stent 12 preferably extends around the entire circumference of the graft and across the opening of the scallop 48, described in further detail below.

Stents at one or both ends of the graft material may be located on the internal surface of at least a portion of the stent graft to provide a smooth outside sealing surface of the graft material against the vessel wall adjacent to the aneurysm as shown in Figure 1. The tubular graft material 14 may be attached to the stent structure 12 through sutures 16 or any other known attachment mechanisms.

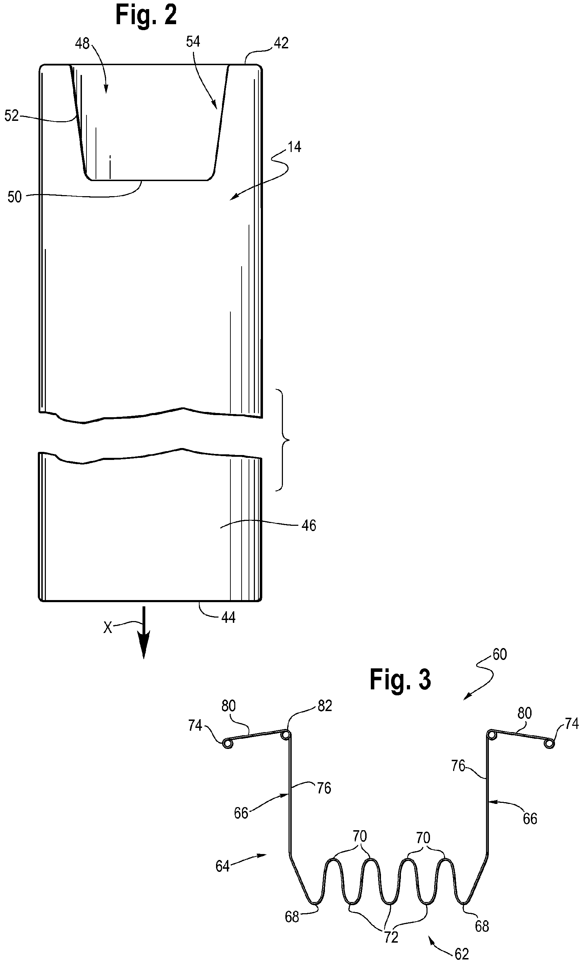

The stent graft 10 includes a proximal end 18 and a distal end 20. As shown in Figure 2, the tubular graft material 14 defines an elongated tubular body extending between the proximal end 18 and the distal end 20. As will be described in greater detail below, the tubular graft material 14 may have one or more scallop-shaped openings or fenestrations 48 (a "scallop") that are cut out of the material of the stent graft 10. It is also contemplated that the stent graft 10 may be bifurcated at one end to accommodate branch vessels such as the iliac arteries.

Figure 2 is a front view of the tubular graft material 14 shown in Figure 1. The tubular graft material 14 may be made of a biocompatible graft material such as polyethylene terephthalate (PET sold under the tradename Dacron®), polyurethane urea optionally blended with a siloxane containing surface modifying additive (such as that sold under the tradename Thoralon®), polytetrafluoroethylene (PTFE) or other synthetic bio-compatible material. A naturally occurring biomaterial, such as collagen, is highly desirable, particularly a specially derived collagen material known as an extracellular matrix (ECM) material, such as small intestinal submucosa (SIS) commercially available from Cook Biotech, West Lafayette, Ind. Besides SIS, examples of ECM's include pericardium, stomach submucosa, liver basement membrane, urinary bladder submucosa, tissue mucosa, and dura mater.

Referring to Figure 2, the tubular graft material 14 includes a proximal edge 42, a distal edge 44, and a tubular body 46 extending therebetween. The tubular graft material 14 may further define a scallop 48 at one or both ends. In one example, the scallop 48 is formed near the proximal end 18 of the stent graft 10 and the scallop 48 opens to the proximal end 18 of the stent graft 10. In another example (not shown), a scallop 48 is located near the distal edge 44 of the stent graft and opens to the distal end 20 of the stent graft 10 and is otherwise similar to the scallop 48 located near the proximal end 18 of the stent graft 10. In one example, the scallop 48 is positioned to allow blood flow into a branch vessel near the site in which the stent graft 10 is placed in the vasculature.

In one example, scallop 48 may be adjacent to the proximal edge 42. As shown in Figures 1-2, the scallop 48 is a cutout portion extending below and distally from the proximal edge 42 of the tubular graft material 14. The scallop 48 is defined by a lateral edge 50 and two opposing longitudinal edges 52. The lateral edge 50 is substantially parallel to the proximal edge 42 of the tubular graft material 14. The opposing longitudinal edges 52 extend generally in a longitudinal direction along the length of the tubular graft material 14 between the proximal edge 42 of the tubular graft material 14 and lateral edge 50 of the scallop 48. The lateral edge 50 and the two opposing longitudinal edges 52 form a perimeter 54 of the scallop 48, which, in one example, defines a generally U-shape configuration. This configuration may expose some of the cells 38 of the proximal stent 22 (as shown in Figure 1).

In some embodiments, the edges 50, 52 and 54 may be substantially straight when the graft material is in a flat configuration, and the edge 50 may be substantially transverse (that is extending perpendicularly) to the longitudinal axis of the graft tubing 14. The skilled person will appreciate that the edges may have other shapes as shown in the drawings.

Referring back to Figure 1, the stent structure 12 may include a proximal sealing stent 22 and one or more distal stents 24. The proximal stent 22 and the one or more distal stents 24 may have the same or different structures. In the embodiment illustrated in Figure 1, the distal stent 24 includes a plurality of first straight struts 26 and a plurality of second straight struts 28 alternately arranged to form a zig-zag ring. The plurality of first and second straight struts 26 and 28 are connected at their opposing ends to form a plurality of connecting ends or apices 30. The tubular graft material 14 may be attached to the distal stent 24 by one or more sutures. In one example, sutures 16 connect the graft material 14 and the distal stent 24 at the apices 30.

The proximal stent 22 may include a plurality of first curved struts 32 and a plurality of second curved struts 34. The first and second curved struts 32 and 34 are alternately arranged to form a generally elliptical or ring-like configuration. The first and second curved struts 32 and 34 are connected at their opposing ends or apices 36 so that one first curved strut 32 and one adjacent second curved strut 34 form a closed cell 38 having an enlarged middle portion and opposing narrowed portions. The closed cells 38 are connected to at least one point along the sides 40 of the first and second curved struts 32 and 34 to form a ring-like configuration. The apices 36 of the first and second curved struts 32 and 34 may be configured to have generally smooth rounded edges to avoid damaging the vessel lumen wall.

Referring back to Figure 1, the opposing longitudinal edges 52 of the scallop 48 may be aligned with a portion of the adjacent first and second curved struts 32 and 34 of the proximal stent 22. In the example shown in Figure 1, the longitudinal edge 52 on the right side of the scallop 48 overlaps a distal portion of the first curved strut 32, whereas the longitudinal edge 52 on the left side of the scallop 48 overlaps a distal portion of the second curved strut 34.

The opposing longitudinal edges 52 of the scallop 48 of the tubular graft material 14 can be stitched by sutures 16 to the first and second curved struts 32 and 34 along at least part of the length of the first and second curved struts 32 and 34. Therefore, the opposing longitudinal edges 52 can conform to a part of the shape of the first and second curved struts 32 and 34.

As described in more detail below, the scallop 48 formed in the tubular graft material 14 may be supported by one or more structures, including a base strut 56 and a support structure 60 that provides additional support for scallop 48. A base strut 56 may be disposed along the lateral edge 50 of the scallop 48. The base strut 56 may be secured to the stent structure 12 and/or the tubular graft material 14 by any known attachment mechanisms. For example, the base strut 56 may be attached to the stent structure 12 and/or the tubular graft material 14 by stitching, gluing, welding, or sutures. In one example, the base strut 56 is sewn along the lateral edge 50 of the scallop 48 to connect the support structure 60 and the tubular graft material 14 at stitch line 15.

The stent graft 10 may further include a support structure 60 to provide additional support for the scallop 48 of the tubular graft material 14. The support structure may be located anywhere on the stent graft 10, and in one example, the support structure is located adjacent to the perimeter of the scallop 48.

The support structure 60 may have any suitable design that preferably corresponds to and/or accommodates the general shape and dimension of the scallop 48. As described below, Figures 1 and 3-7 show several embodiments of support structures that can be used to support scallop 48. In one example, the support structure 60 shown in Figure 1 (and described in greater detail below in connection with Figure 3) may have a series of zig-zag-shaped segments located distal to the lateral edge 50 of the scallop 48.

The support structure 60 may be secured to the stent structure 12 and/or the tubular graft material 14 by any known attachment mechanisms. For example, the support structure 60 may be attached to the stent structure 12 and/or to the tubular graft material 14 by stitching, gluing, welding, or sutures. In one example, the support structure 60 is sewn onto the graft material 14 along one or more points of the longitudinal edges 52 of scallop 48. The support structure 60 may also be sewn onto the graft material 14 just distal to the lateral edge 50 of the scallop 48.

The support structures described herein may be formed from any biocompatible material such as stainless steel, nickel-titanium alloy (nitinol), ceramic, cobalt/chromium alloys, aluminum or other biocompatible metals and/or composites or alloys such as carbon or carbon fiber. In one example, the support structures are made of stainless steel and are resilient, which allows the stent graft 10 to be delivered to a target site in a compressed configuration and to expand in a deployment configuration.

The support structure 60 (and other support structures disclosed below) may be configured to support the graft material 14. The support structure may also serve to reduce tearing or separation of the graft material 14 from the stent structure 12. In one example, the support structure 60 reinforces the scallop 48 so that blood flow forces do not tear, separate, or disfigure the graft material and/or push the graft material 14 away from or off of the stent structure 12.

In the case of balloon expandable stents, the support structure 60 (and other support structures disclosed below) may provide a more dimensionally stable scallop 48 in the tubular graft material 14, particularly when a balloon expandable stent is used as a leg or side arm extension through a branch vessel (not shown). With the support structure 60, the force of the balloon expansion is less likely to tear the graft material 14 from the stent structure 12.

Figure 3 illustrates the support structure shown in combination with the stent graft of Figure 1. Referring to Figure 3, the support structure 60 includes a lateral base 62 and an attaching member 64. The lateral base 62 includes distal ends 68, a plurality of proximal bends 70 and a plurality of distal bends 72 disposed between the distal ends 68. The lateral base 62 comprises a zig-zag configuration. The attaching member 64 includes a pair of opposing struts 66, which extend from the distal ends 68 of the lateral base 62. The free ends of the opposing struts 66 are formed as a loop 74 to prevent the sharp edges of the opposing struts 66 from damaging the lumen wall.

The support structure 60 may be attached to the stent structure 12 or to the tubular graft material 14, or both. In one example, the lateral base 62 is disposed along the lateral edge 50 of the scallop 48, and as illustrated in Figure 1, lateral base 62 is disposed distal to the lateral edge 50. No portion of the lateral base 62 protrudes into the opening formed by scallop 48, such that insertion of an extension leg (not shown), if needed, is not blocked and the blood flow through scallop 48 is not obstructed. The plurality of proximal bends 70 are closer to the lateral edge 50 of the scallop 48 than the distal bends 72. The opposing struts 66 of the support structure 60 are disposed generally along the opposing longitudinal edges 52 of the scallop 48.

A portion of the support structure 60 may have a shape that generally conforms to the shape of at least a portion of the first or second curved struts 32 and 34 of stent structure 12. As an example shown in Figure 3, the opposing struts 66 may have a first segment 76 that generally conforms to the contour of a distal portion of the adjacent first and second curved struts 32 and 34 and a second segment 80 that generally conforms to the contour of the proximal edge 42 of the stent graft material 14. A loop 82 may be formed between the first segment 76 and the second segment 80.

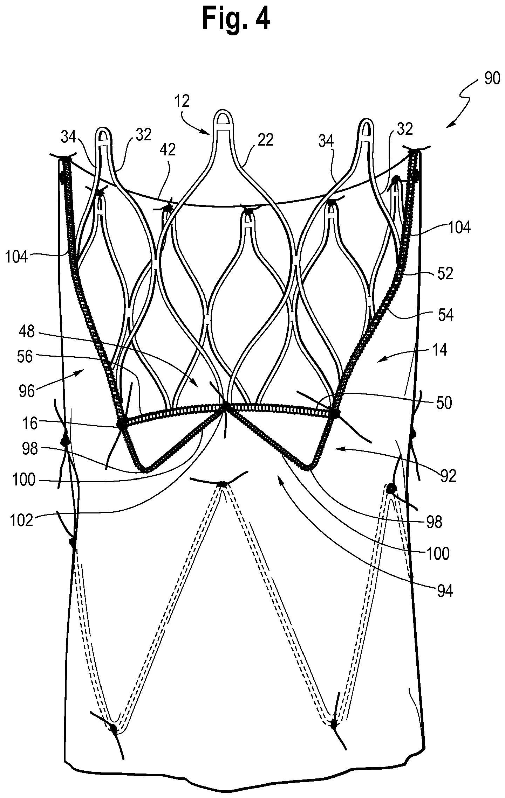

Figure 4 is a perspective view of a stent graft 10 constructed in accordance with a second embodiment of the present disclosure. Referring to Figure 4, a stent graft 90 has a structure similar to that of the stent graft 10 of Figure 1, except the support structure 60 has a different shape. Therefore, similar reference numerals are used in Figure 4 for similar components to those in Figure 1.

The stent graft 90 includes a stent structure 12, a tubular graft material 14, and a support structure 92. Similar to the first embodiment, scallop 48 is formed in the tubular graft material 14 having a perimeter 54 including a lateral edge 50 and opposing longitudinal edges 52. At least a portion of the opposing longitudinal edges 52 extend along a portion of the first and second curved struts 32 and 34.

A base strut 56 is disposed along the lateral edge 50 of the scallop 48. The perimeter 54 of the scallop 48 is attached at one or more points to the first and second curved struts 32, 34 and the base strut 56.

The support structure 92 includes a lateral base 94 and an attaching member 96. The lateral base 94 includes one or more bent segments 98 and an apex 102 between the one or more bent segments 98 that is adjacent to the base strut 56. The attaching member 96 includes a pair of opposing struts104 extending from the lateral base 94 and disposed generally along the longitudinal edges 52 of the scallop 48. The opposing struts 104 may be attached at one or more points along a portion of the first and second curved struts 32 and 34 of the proximal stent 22. In one example, the opposing struts 104 may also include a first segment 76 and a second segment 80 similar to the opposing struts 66 in Figure 3.

Similar to support structure 60, the support structure 92 may be secured to the stent structure 12 and/or the tubular graft material 14 by any known attachment mechanism. For example, the support structure 92 may be attached to the stent structure 12 and/or the tubular graft material 14 by stitching, gluing, welding, or sutures. In one example shown in Figure 4, the support structure 92 is sutured along the perimeter 54 of the scallop 48 and just distal to the scallop 48 to connect the support structure 92 and the tubular graft material 14 at sutures 16.

The support structure 92 may be formed from any biocompatible material such as stainless steel, nickel-titanium alloy (nitinol), ceramic, cobalt/chromium alloys, aluminum or other biocompatible metals and/or composites or alloys such as carbon or carbon fiber. In one example, the support structure 92 is made of stainless steel and is resilient, which allows the scallop 48 and the stent graft 90 to be delivered in a compressed configuration and to expand in a deployment configuration.

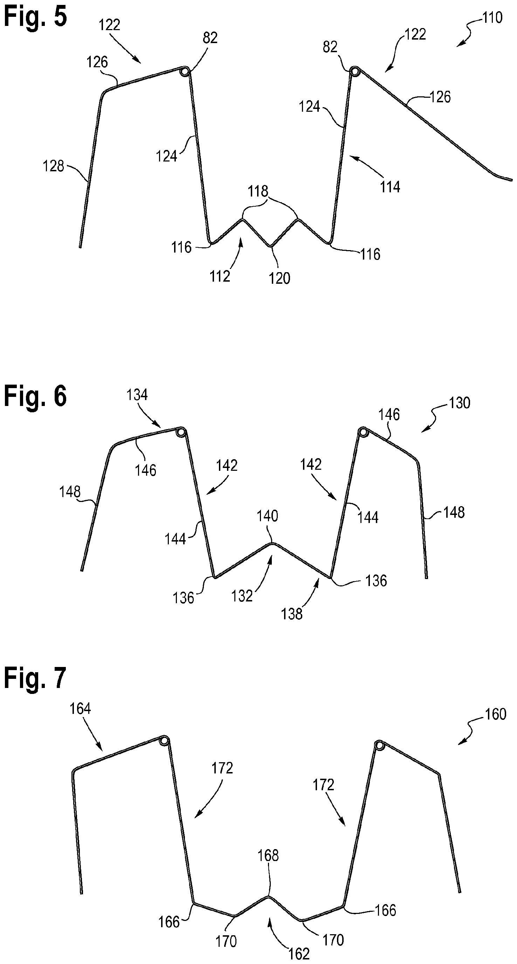

Figure 5 is a view of a support structure 110 for a stent graft in accordance with a third embodiment of the present disclosure. Referring to Figure 5, a support structure 110 can be used with any suitable stent graft, including stent grafts 10 and 90 shown in Figures 1 and 4. The support structure 110 includes a lateral base 112 and an attaching member 114. The lateral base 112 defines one or more bent segments 116, a plurality of proximal bends or apices 118, and a distal bend or apex 120 between the one or more bent segments 116. In one example, the lateral base 112 defines a zig-zag configuration. The attaching member 114 includes a pair of opposing struts 122 attached to the one or more bent segments 116.

The opposing struts 122 each include a first segment 124 connected to the one or more bent segments 116 of the lateral base 112, and a second segment 126 connected to an end of the first segment 124. A loop 82 may be formed between the first segment 124 and the second segment 126. One or both of the second segments 126 may further include a third segment 128 connected to the second segment 126. The opposing struts may or may not be symmetrical. For example, as shown in Figure 5, the opposing struts 122 may not be symmetrical in that only one of the opposing struts 122 has a first segment 124, second segment 126, and a third segment 128. The other of the opposing struts 122 only has a first segment 124 and a second segment 126.

The support structure 110 may be attached to the stent structure 12 and/or the graft material 14. In one example, the first segments 124 may be attached to the graft material 14 along the opposing longitudinal edges 52 of the scallop 48 and/or to at least a portion of the first and second curved struts 32 and 34. The second segment 126 may be disposed along at least a portion proximal edge 42 of the tubular graft material 14. The third segment 128 may be attached along a portion of the first and second curved struts 32 and 34.

The three segments 124, 126 and/or 128 shown in Figure 5 may be applied to the other support structures in the disclosure. In other words, any of the other disclosed support structures may be modified so that the opposing struts 122 have three segments.

Figure 6 is a view of a support structure 130 for a stent graft 10 in accordance with a fourth embodiment of the present disclosure. Referring to Figure 6, a support structure 130 can be used with any suitable stent graft, including stent grafts 10 and 90 shown in Figures 1 and 4. The support structure 130 includes a lateral base 132 and an attaching member 134. The lateral base 132 includes one or more bent segments 136, and one or more distal bends or apices 138 and at least one proximal apex 140. The attaching member 134 includes a pair of opposing struts 142 extending from the one or more bent segments 136 of the lateral base 132. The opposing struts 142 each include a first segment 144, a second segment 146, and a third segment 148. Figure 7 is a view of a support structure 160 for a stent graft in accordance with a fifth embodiment of the present disclosure. Referring to Figure 7, a support structure 160 can be used with any suitable stent graft, including stent grafts 10 and 90 shown in Figures 1 and 4. The support structure 160 includes a lateral base 162 and an attaching member 164. The lateral base 162 includes one or more bent segments 166, a proximal bend or apex 168 and two distal bends or apices 170. The distal apices 170 may each define an obtuse angle.

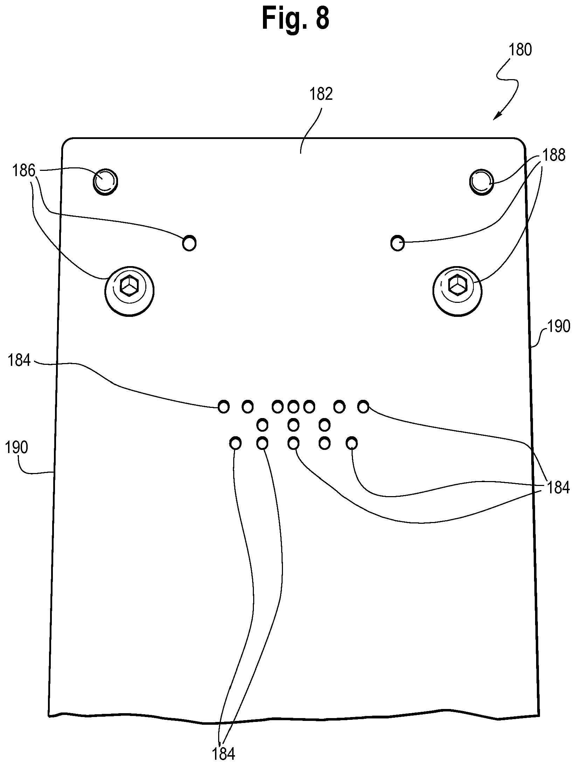

The attaching member 164 includes a pair of opposing struts 172 extending from the lateral base 162. Figure 8 is a schematic view of a setting fixture for manufacturing a support structure in accordance with the teachings of the present disclosure.

The setting fixture 180 shown in Figure 8 may be used for manufacturing the support structures 60, 92, 110, 130, and 160 used to enforce the scallop 48 of a stent graft. The setting fixture 180 may include a plate body 182 and a plurality of first apertures 184, a plurality of second apertures 186, and a plurality of third apertures 188 through the plate body 182. In one example, the first apertures 184 are located near the center of the plate body 182. The first apertures 184 may be in any arrangement, including in a row or plurality of rows. In one example, the second apertures 186 are located near an edge or corner of the plate 182 and the third apertures 188 are located at an adjacent edge or corner of the plate 182.

The plurality of first apertures 184, second apertures 186 and third apertures 188 may have any number of openings in the plate body 182. In one example, the plurality of first apertures 184 may have 15 openings, the plurality of second apertures 186 may have 3 openings and the plurality of third apertures 188 may have 3 openings in the plate body 182.

The first apertures 184 may be used to set the shape of the lateral base of the support structure, whereas the second apertures 186 and the third apertures 188 may be used to set the two opposing struts of the attaching member of the support structure.

The support structure according to any of the embodiments in this disclosure may be formed by securing one or more wires to the plate body 182 at locations corresponding to one or more of the first, second and third apertures 184, 186, 188 to set the shape of the support structure. In one example, a single wire is used to create the support structure. The shape of the lateral base of the support structure may be set by the first apertures 184 by securing a portion of the wire to the first apertures 184 at predetermined locations. The lateral base may be set to have any shape, including a linear shape, a zig-zag shape, a single curve shape, an arc shape, a triangular shape, or a wavy shape. The lateral base can have any other shape as long as it is disposed distally of the bottom edge of the scallop 48 to provide additional support for the scallop 48. The shape of the opposing struts of the attaching member of the support structure may be set by the second and third apertures 186 and 188 by securing another portion of the wire to the second apertures 186 and the third apertures 188, respectively, at predetermined locations.

After the wire is attached to the setting fixture 180, the wire may be subject to heat treatment to set the configuration of the support structure. After the support structure is formed, the support structure may be attached to any existing stent graft to provide additional support for a scallop 48.

It should be noted that the disclosure is not limited to the embodiment described and illustrated as examples.

## Claims

1. A stent graft (10, 90) comprising:
a substantially tubular graft (14) having a first end (18) and a second end (20);
a scallop cut out (48) of one or both of the first end (18) and the second end (20), wherein the scallop (48) extends away from an end edge (42) of the substantially tubular graft (14) and has a perimeter defined by graft material, wherein the perimeter has at least three sides including a lateral side (50) having a lateral edge substantially parallel to the end edge and two longitudinal sides (52, 54) extending between the lateral side and the end edge; and
a scallop support structure (60, 92,110,130,160) at least partially bounded about the perimeter of the scallop (48), the scallop support structure comprising at least two substantially longitudinal perimeter support sections (66,124,144,172), and an undulating lateral base (62,94,112,132,162) extending between the at least two substantially longitudinal perimeter support sections, wherein the undulating lateral base has at least two undulations;
wherein one of the at least two perimeter support sections is attached along and defines one of the two longitudinal sides and another of the at least two perimeter support sections is attached along and defines the other of the longitudinal sides; and
wherein the undulating lateral base is disposed along the lateral base of the scallop (48) and overlaps the graft material.

2. A stent graft according to claim 1, wherein the undulations of the undulating lateral side comprise a plurality of peaks (70,102,118,140,168) and a plurality of valleys (72,98,120,136,170) and the plurality of peaks abut the lateral edge of the lateral side and the plurality of valleys extend distally away from the lateral edge of the scallop.

3. A stent graft according to claim 1 or 2, wherein the undulations of the undulating lateral base comprise at least one peak abutting the lateral base and at least two valleys extending away from the lateral edge of the scallop.

4. A stent graft according to any preceding claim, wherein the scallop support structure is formed of a single continuous wire.

5. A stent graft according to any preceding claim, wherein the undulations of the undulating lateral base comprise:
a series of U-shaped undulations, and preferably wherein end edges of the U-shaped undulations abut the edge of the lateral side; or
a series of V-shaped undulations, and preferably wherein end edges of the V-shaped undulations abut the edge of the lateral side.

6. A stent graft according to any preceding claim, comprising a support wire (56), separate from the undulating lateral base, wherein the support wire bounds at least the length of the edge of the lateral side and is coincident with the edge of the lateral side.

7. A stent graft according to any preceding claim, wherein the first end (42) of the graft is the proximal end of the graft and the second end (44) of the graft is the distal end of the graft.

8. A stent graft according to any preceding claim, comprising a plurality of stents (24) along a length of the tubular graft (14) from the first end to the second end, wherein the scallop support structure is a separate structural element from any of the plurality of stents.

9. A stent graft according to any preceding claim, wherein the scallop support structure comprises a second lateral base that extends along the lateral end to define the lateral edge.

## Patentansprüche

1. Stentgraft (10, 90), umfassend:
eine im Wesentlichen röhrenförmige Prothese (14) mit einem ersten Ende (18) und einem zweiten Ende (20);
einen bogenförmigen Ausschnitt (48) eines oder beider des ersten Endes (18) und des zweiten Endes (20), wobei sich die Bogenform (48) von einem Endrand (42) der im Wesentlichen röhrenförmigen Prothese (14) weg erstreckt und einen vom Graftmaterial definierten Umfang aufweist, wobei der Umfang mindestens drei Seiten aufweist, einschließlich einer lateralen Seite (50) mit einem Seitenrand im Wesentlichen parallel zum Endrand und zwei Längsseiten (52, 54), die sich zwischen der lateralen Seite und dem Endrand erstrecken; und
eine Bogenformstützstruktur (60, 92, 110, 130, 160), die zumindest teilweise um den Umfang der Bogenform (48) begrenzt ist, wobei die Bogenformstützstruktur mindestens zwei im Wesentlichen längliche Umfangsstützabschnitte (66, 124, 144, 172) und eine sich zwischen den mindestens zwei im Wesentlichen länglichen Umfangsstützabschnitten erstreckende wellenförmige Seitenbasis (62, 94, 112, 132, 162) umfasst, wobei die wellenförmige Seitenbasis mindestens zwei Wellenformen aufweist;
wobei einer der mindestens zwei Umfangsstützabschnitte entlang einer der zwei Längsseiten befestigt ist und diese definiert und ein anderer der mindestens zwei Umfangsstützabschnitte entlang der anderen der Längsseiten befestigt ist und diese definiert; und
wobei die wellenförmige Seitenbasis entlang der Seitenbasis der Bogenform (48) angeordnet ist und das Graftmaterial überlappt.

2. Stentgraft nach Anspruch 1, wobei die Wellenformen der wellenförmigen lateralen Seite eine Vielzahl von Spitzen (70, 102, 118, 140, 168) und eine Vielzahl von Mulden (72, 98, 120, 136, 170) umfassen und die Vielzahl von Spitzen an den Seitenrand der lateralen Seite anstoßen und die Vielzahl von Mulden sich distal vom Seitenrand der Bogenform weg erstrecken.

3. Stentgraft nach Anspruch 1 oder 2, wobei die Wellenformen der wellenförmigen Seitenbasis mindestens eine Spitze umfassen, die an die Seitenbasis anstößt, und mindestens zwei Mulden, die sich vom Seitenrand der Bogenform weg erstrecken.

4. Stentgraft nach einem der vorhergehenden Ansprüche, wobei die Bogenformstützstruktur aus einem einzelnen durchgehenden Draht geformt ist.

5. Stentgraft nach einem der vorhergehenden Ansprüche, wobei die Wellenformen der wellenförmigen Seitenbasis das Folgende umfassen:
eine Reihe von U-förmigen Wellenformen, und vorzugsweise wobei Endränder der U-förmigen Wellenformen an den Rand der lateralen Seite anstoßen; oder
eine Reihe von V-förmigen Wellenformen, und vorzugsweise wobei Endränder der V-förmigen Wellenformen an den Rand der lateralen Seite anstoßen.

6. Stentgraft nach einem der vorhergehenden Ansprüche, umfassend einen Stützdraht (56), der von der wellenförmigen Seitenbasis getrennt ist, wobei der Stützdraht zumindest die Länge des Rands der lateralen Seite begrenzt und mit dem Rand der lateralen Seite zusammenfällt.

7. Stentgraft nach einem der vorhergehenden Ansprüche, wobei das erste Ende (42) der Prothese das proximale Ende der Prothese ist und das zweite Ende (44) der Prothese das distale Ende der Prothese ist.

8. Stentgraft nach einem der vorhergehenden Ansprüche, umfassend eine Vielzahl von Stents (24) entlang einer Länge der röhrenförmigen Prothese (14) vom ersten Ende zum zweiten Ende, wobei die Bogenformstützstruktur ein von einem beliebigen der Vielzahl von Stents gesondertes Strukturelement ist.

9. Stentgraft nach einem der vorhergehenden Ansprüche, wobei die Bogenformstützstruktur eine zweite Seitenbasis umfasst, die sich entlang des Seitenendes erstreckt, um den Seitenrand zu definieren.

## Revendications

1. Greffon de stent (10, 90) comprenant :
un greffon sensiblement tubulaire (14) ayant une première extrémité (18) et une seconde extrémité (20) ;
une découpe de feston (48) de la première extrémité (18) et/ou de la seconde extrémité (20), le feston (48) s'étendant à l'écart d'un bord d'extrémité (42) du greffon sensiblement tubulaire (14) et ayant un périmètre défini par un matériau de greffon,
le périmètre ayant au moins trois côtés compris un côté latéral (50) ayant un bord latéral sensiblement parallèle au bord d'extrémité et deux côtés longitudinaux (52, 54) s'étendant entre le côté latéral et le bord d'extrémité ; et
une structure de support de feston (60, 92, 110, 130, 160) délimitée au moins partiellement autour du périmètre du feston (48), la structure de support de feston comprenant au moins deux sections de support de périmètre sensiblement longitudinales (66, 124, 144, 172), et une base latérale ondulante (62, 94, 112, 132, 162) s'étendant entre les au moins deux sections de support de périmètre sensiblement longitudinales, la base latérale ondulante ayant au moins deux ondulations ;
l'une des au moins deux sections de support périphérique étant fixée le long d'un des deux côtés longitudinaux et le définissant et une autre des au moins deux sections de support périphérique étant fixée le long de l'autre des côtés longitudinaux et le définissant ; et
la base latérale ondulante étant disposée le long de la base latérale du feston (48) et recouvrant le matériau de greffon.

2. Greffon de stent selon la revendication 1, les ondulations du côté latéral ondulant comprenant une pluralité de pics (70, 102, 118, 140, 168) et une pluralité de vallées (72, 98, 120, 136, 170), et la pluralité de pics venant en butée contre le bord latéral du côté latéral, et la pluralité de vallées s'étendant à l'écart du bord du feston.

3. Greffon de stent selon la revendication 1 ou 2, les ondulations de la base latérale ondulante comprenant au moins un pic venant en butée contre la base latérale et au moins une vallée s'étendant à l'écart du bord latéral du feston.

4. Greffon de stent selon n'importe quelle revendication précédente, la structure de support du feston étant formée d'un seul fil continu.

5. Greffon de stent selon n'importe quelle revendication précédente, les ondulations de la base latérale ondulante comprenant :
une série d'ondulations en forme de U, et de préférence les bords d'extrémité des ondulations en forme de U venant en butée contre le bord du côté latéral ; ou
une série d'ondulations en forme de V, et de préférence les bords d'extrémité des ondulations en forme de V venant en butée contre le bord du côté latéral.

6. Greffon de stent selon n'importe quelle revendication précédente, comprenant un fil de support (56), séparé de la base latérale ondulante, le fil de support délimitant au moins la longueur du bord du côté latéral et coïncidant avec le bord du côté latéral.

7. Greffon de stent selon n'importe quelle revendication précédente, la première extrémité (42) du greffon étant l'extrémité proximale du greffon et la seconde extrémité (44) du greffon étant l'extrémité distale du greffon.

8. Greffon de stent selon n'importe quelle revendication précédente, comprenant une pluralité de stents (24) le long d'une longueur du greffon tubulaire (14) de la première extrémité à la seconde extrémité, la structure de support de feston étant un élément structurel séparé de l'un quelconque de la pluralité de stents.

9. Greffon de stent selon n'importe quelle revendication précédente, la structure de support du feston comprenant une seconde base latérale qui s'étend le long de l'extrémité latérale pour définir le bord latéral.
